# EUROPÄISCHE PATENTANMELDUNG

(11) **EP 1 419 734 A1**
(43) Veröffentlichungstag der Anmeldung: **19.05.2004**
(21) Anmeldenummer: 02025311.8
(22) Anmeldetag: 13.11.2002
(51) Int. Cl.: A61B 5/103

(54) **Belastungsüberwacher für den menschlichen Bewegungsapparat**

(71) Anmelder: Fritz Minke GmbH & Co. KG, 47051 Duisburg (DE)
(72) Erfinder: Minke-Korsinnek, Christiane, 47051 Duisburg (DE)
(74) Vertreter: Stenger, Watzke & Ring Patentanwälte

(57) **Zusammenfassung**

Um eine Vorrichtung zur Überwachung der auf einem Bein aufgebrachten Belastung, mit einem Drucksensor (2), einer Auswerte- und Anzeigeeinheit (1) sowie einer zwischen beiden wirkenden Datenverbindung (3) bereitzustellen, welche geeignet ist, für medizinische bzw. therapeutische Zwecke wirksam Fehl- und Überbelastungen zu signalisieren, wird mit der Erfindung vorgeschlagen, daß die Auswerte- und Anzeigeeinheit einen Speicher für wenigstens einen Belastungsgrenzwert sowie eine Steuerung zur Meßwertaufnahme, einem Vergleich des Meßwertes mit dem Belastungsgrenzwert und einer Signalabgabe bei Überschreiten des Belastungsgrenzwertes durch den Meßwert umfaßt.

## Beschreibung

Die vorliegende Erfindung betrifft eine Vorrichtung zur Überwachung der auf einem Bein aufgebrachten Belastung, mit einem Drucksensor, einer Auswerte- und Anzeigeeinheit sowie einer zwischen beiden wirkenden Datenverbindung.

Vorrichtungen zur Überprüfung und Überwachung der auf Gliedmaßen wirkenden Belastungen werden in unterschiedlichen Bereichen benötigt. Dazu gehören die Überprüfung oder Kontrolle der postoperativen Teilbelastungen, beispielsweise nach Beinbrüchen, Teilprothesen- oder Implantateinsatz, im Bereich der Prothetik zur Verhinderung von Vermeidungsverhalten, zur Gangkontrolle beispielsweise nach Schlaganfällen, bei Sehbehinderungen, Lähmungen und dergleichen, bei Diabetes als Ersatz für fehlende Sensorik und Schmerzempfinden sowie beispielsweise bei einem Muskelaufbau durch Belastungstraining. Darüber hinaus sind eine Vielzahl von Anwendungsfällen denkbar, beispielsweise Hüftkorrektur und dergleichen.

In allen genannten Fällen geht es darum, Überbelastungen zu vermeiden.

Aus der DE 38 27 999 ist eine Meßvorrichtung bekannt, bei welcher über eine große Fläche eine Vielzahl von Sensoren positioniert sind. Diese dienen dazu, die unterschiedlichen Kräfte zu erfassen, damit festgestellt werden kann, ob eine betroffene Person beispielsweise wegen auftretender Schmerzen oder dergleichen Belastungen zu vermeiden versucht. Eine gattungsgemäße Vorrichtung ist aus der DE 41 35 940 bekannt, bei welcher jedoch die Einstellung eines Druckgrenzwertes kompliziert ist und dieser insbesondere während einer Nutzungsperiode nicht variiert werden kann. Die EP 0 335 467 beschreibt einen Turnschuh, bei welchem aufgrund austretender Druckkräfte im Sohlenbereich optische Signale im Schuh erzeugt werden. Vergleichbare Vorrichtungen sind bekannt aus der GB 2 263 386 und beispielsweise in einem Kinderschuh zur Abgabe eines akustischen Signals aus der US-PS 5 402 590 sowie dem DE-GM 79 30 576 und der US-PS 5 421 107.

Keine der vorbekannten Vorrichtungen ermöglicht einen sinnvollen Einsatz im medizinisch-/therapeutischen Bereich zur Vermeidung von Überbelastungen.

Insbesondere sind keinerlei Vorrichtungen bekannt, die den Ansprüchen an eine ernsthafte therapeutische oder medizinische Kontrolle genügen. Auch sind keine Vorrichtungen bekannt, mit denen es möglich ist, Belastungsprofile einzustellen oder im nachhinein beispielsweise aus therapeutischen Gründen den Belastungsverlauf zu überprüfen. In bezug auf die Genauigkeit und vor allem Reaktionsschnelle erfüllen bekannte Vorrichtungen die erforderlichen Bedingungen nicht.

Ausgehend vom vorbeschriebenen Stand der Technik liegt der Erfindung die **Aufgabe** zugrunde, eine Vorrichtung zur Überwachung der auf einem Bein aufgebrachten Belastung, mit einem Drucksensor, einer Auswerte- und Anzeigeeinheit sowie einer zwischen beiden wirkenden Datenverbindung bereitzustellen, welche geeignet ist, für medizinische bzw. therapeutische Zwecke wirksam Fehlund Überbelastungen zu signalisieren.

Zur technischen **Lösung** dieser Aufgabe wird mit der Erfindung vorgeschlagen, daß die Auswerte- und Anzeigeeinheit einen Speicher für wenigstens einen Belastungsgrenzwert sowie eine Steuerung zur Meßwertaufnahme, einem Vergleich des Meßwertes mit dem Belastungsgrenzwert und einer Signalabgabe bei Überschreiten des Belastungsgrenzwertes durch den Meßwert umfaßt.

Mit der erfindungsgemäßen Vorrichtung ist es nunmehr möglich, individuell einen Belastungsgrenzwert für die jeweilige Person und Situation festzulegen und in die Vorrichtung einzuspeichern, so daß, wenn auf den Belastungssensor ein den Belastungsgrenzwert überschreitender Druck aufgebracht wird, ein Signal erzeugt wird. Durch diesen Effekt können Überbelastungen ermittelt werden, so daß sich bei der betroffenen Person ein Lerneffekt einstellt, der zur Vermeidung von Überbelastungen führt.

Der Belastungssensor wird unter der Fußsohle positioniert, während die Auswerteund Anzeigeeinheit an beliebiger Stelle am Körper, am Bein, am Gürtel oder einer anderen geeigneten Position angeordnet werden kann. Die Datenverbindung zwischen beiden kann ein Kabel oder auch eine drahtlose Verbindung sein. Im letzteren Fall ist der Sensor noch mit einer Energiequelle zu versehen.

Die Signalisierung kann auf unterschiedliche Weise erfolgen, beispielsweise akustisch, optisch oder sensorisch. Auch Kombinationen sind möglich. Je nach Krankheitsprofil der betroffenen Person kann die geeignete Anzeige festgelegt werden.

In vorteilhafter Weise kann die Steuerung mit einer signalen Echtzeit verarbeitenden Schaltung versehen sein. Hier können geeignete Rechnerchips im Zusammenwirken mit schnellen Speichern Sofortanzeigen bewirken, sobald Überlastungen auftreten.

Gemäß einem besonders vorteilhaften Vorschlag der Erfindung kann der Belastungsgrenzwert dadurch erfaßt und gespeichert werden, daß auf den Belastungssensor bei eingeschaltetem Gerät der entsprechende Druck ausgeübt und die Steuerung zur Aufnahme des aktuell gemessenen Druckwertes veranlaßt wird. Dies ermöglicht, daß zusätzlich zu den mechanisch/medizinischen Größen auch individuelle Faktoren wie Schmerzempfinden und dergleichen berücksichtigt werden.

Gemäß einem weiteren vorteilhaften Vorschlag der Erfindung ist vorgesehen, daß ein zeitbezogenes Belastungsprofil in den vorrichtungsseitigen Speicher eingespeichert und durch die Steuerung überprüft wird. Bei vielen Anwendungen kommt es nicht nur darauf an, bestimmte Belastungswerte gänzlich zu vermeiden, sondern auch darauf, daß bestimmte Belastungen nur innerhalb von Zeitgrenzen auftreten dürfen, also eine bestimmte vorgegebene Belastung nicht zu lange auftreten darf. Derartige Belastungsprofile können beispielsweise von den Medizinern oder Therapeuten auf Rechnern festgelegt und über eine gemäß einem vorteilhaften Vorschlag an der Auswerte-/Anzeigeeinheit angeordnete Computerschnittstelle in das Gerät eingespielt werden.

Ein weiterer vorteilhafter Vorschlag der Erfindung sieht vor, daß das tatsächlich auftretende Belastungsprofil im Gerät abrufbar gespeichert wird, zumindest aber Überlastungen hinsichtlich ihres qualitativen Wertes gespeichert werden. In diesen Fällen können die Therapeuten über die Computerschnittstelle die Werte auslesen und sich gegebenenfalls graphisch über die Zeit darstellen lassen. Sind nur Überschreitungen der Belastungsgrenzwerte als reiner Belastungswert gespeichert, hat der Therapeut zumindest einen Einblick in den Belastungszustand des Beines.

Mit der Erfindung wird eine mit geringem wirtschaftlichen Aufwand erstellbare aber einen großen medizinischen und therapeutischen Effekt erzielende Vorrichtung vorgeschlagen, die betroffene Personen bei der Vermeidung von Überlastungen unterstützt und ihnen ermöglicht, ein Gefühl für die Belastungsgrenzen zu entwikkeln.

Weitere Vorteile und Merkmale ergeben sich aus der folgenden Beschreibung anhand der Figuren. Dabei zeigen:

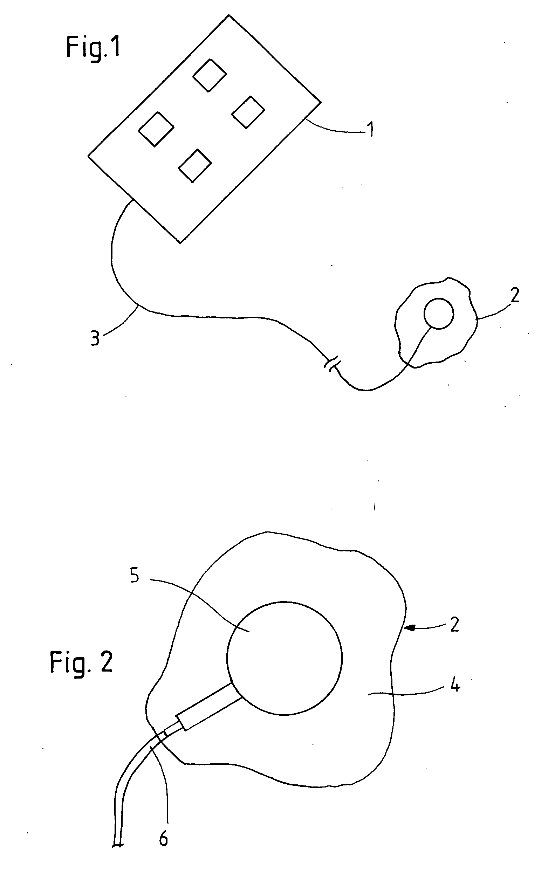

- Fig. 1: eine Darstellung eines Ausführungsbeispiels für eine erfindungsgemäße Vorrichtung;
- Fig. 2: eine vergrößerte Darstellung des Drucksensors gemäß Fig. 1 und

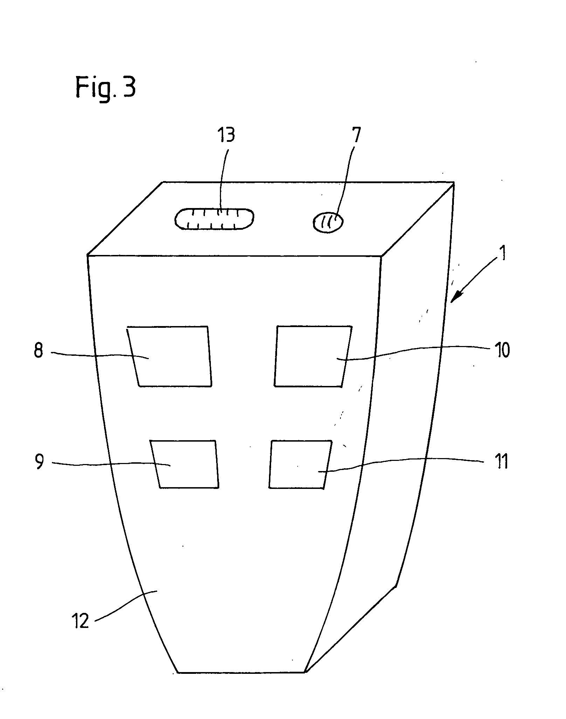

- Fig. 3: eine vergrößerte Darstellung der Auswerte-/Anzeigeeinheit gemäß Fig. 1.

In den Fig. sind gleiche Elemente mit gleichen Bezugszeichen versehen. Die Gesamteinheit umfaßt eine beispielsweise mittels eines nicht gezeigten Clips an einem Gürtel oder einem Beinriemen befestigbare Bedien- und Anzeigeeinheit 1, welche im gezeigten Ausführungsbeispiel mit einem Kabel 3 mit einer Drucksensoreinheit 2 verbunden ist.

Die Drucksensoreinheit 2 umfaßt ein vorzugsweise elastisches Trägerelement 4 aus Silikon oder dergleichen sowie einen Drucksensor 5. Der Drucksensor 5 ist so in das Trägerelement 4 eingelagert, daß das Trägerelement auftretende Brücke durch Dämpfung nicht verfälscht, bzw. werden die Dämpfungseigenschaften etwaiger unterlagernder Schichten in einer in der Bedien- und Anzeigeeinheit 1 enthaltenen Steuereinheit kompensiert.

Die Bedien- und Anzeigeeinheit 1 umfaßt ein Gehäuse 12, in welchem an geeigneter Stelle eine Steckbuchse 7 angeordnet ist. Eine am Drucksensor 5 ausgebildete Anschlußstelle 6 kann dann über ein Kabel 3, dessen Ende mit einem geeigneten Stecker versehen ist, mit der Bedien- und Anzeigeeinheit verbunden werden.

Im gezeigten Ausführungsbeispiel sind Tasten 8, 9, 10 und 11 ausgebildet, welche zur Bedienung vorgesehen sind. So kann beispielsweise beim Aufbringen eines gewünschten Druckes auf den Drucksensor 5 durch Drücken einer der Tasten der Druckgrenzwert erfaßt und gespeichert werden. Eine der Tasten kann zur Einstellung verschiedener Modi vorgesehen sein. Eine weitere Taste kann als Ein-/Aus-Taste ausgelegt sein. Über eine weitere Taste kann beispielsweise der Anzeige- bzw. Signalmodus ausgewählt werden, beispielsweise ein Vibrationsalarm ein- bzw. ausgeschaltet werden, eine akustische Signaleinheit zugeschaltet oder abgeschaltet werden und dergleichen. Optional können Bedienelemente zur Einstellung von Lautstärken, Vibrationsintensitäten und dergleichen vorgesehen sein. Im gezeigten Ausführungsbeispiel ist eine Standardschnittstelle 13 am Gehäuse 12 angeordnet, um die Bedien- und Anzeigeeinheit 1 mit einem Computer zu verbinden. Es handelt sich beispielsweise um eine RS232-Schnittstelle. Optional können aber auch Speicherkarten, Speichersticks und dergleichen vorgesehen sein, die über entsprechende Einschübe und Schnittstellen mit der nicht gezeigten Steuereinheit verbunden sind.

Im Betrieb wird die Sensoreinheit 2 an einer geeigneten Stelle, beispielsweise unter einer Fußsohle, in einem orthopädischen Schuh, in einem Gips oder dergleichen positioniert. Über das Kabel 3 wird die Sensoreinheit 2 mit der Anzeige- und Bedieneinheit verbunden und diese entsprechend eingestellt und an einer geeigneten Stelle, an einem Gürtel oder dergleichen positioniert. Bewegt sich nun die Bedienperson, treten entsprechende Drücke auf und werden von der Steuereinheit ausgewertet. Überschreiten die Drücke vorgespeicherte Grenzwerte, kann ein Signal abgegeben werden. Darüber hinaus können Werte und Zeiten optional gespeichert werden. Über die Nutzungszeit kann der Grenzwert auf einfache Weise nachgestellt werden.

Das beschriebene Ausführungsbeispiel dient nur der Erläuterung und ist nicht beschränkend.

### Bezugszeichenliste

- 1: Bedien- und Anzeigeeinheit
- 2: Drucksensoreinheit
- 3: Kabel
- 4: Träger
- 5: Drucksensor
- 6: Kabelanschluß
- 7: Steckbuchse
- 8: Taste
- 9: Taste
- 10: Taste
- 11: Taste
- 12: Gehäuse
- 13: Schnittstelle

## Patentansprüche

1. Vorrichtung zur Überwachung der auf einem Bein aufgebrachten Belastung, mit einem Drucksensor, einer Auswerte- und Anzeigeeinheit sowie einer zwischen beiden wirkenden Datenverbindung, wobei die Auswerte- und Anzeigeeinheit einen Speicher für wenigstens einen Belastungsgrenzwert sowie eine Steuerung zur Meßwertaufnahme, einem Vergleich des Meßwertes mit dem Belastungsgrenzwert und einer Signalabgabe bei Überschreiten des Belastungsgrenzwertes durch den Meßwert umfaßt.

2. Vorrichtung nach Anspruch 1, **dadurch gekennzeichnet, daß** dieser ein Tastenfeld aufweist.

3. Vorrichtung nach einem der vorhergehenden Ansprüche, **dadurch gekennzeichnet, daß** diese eine Computerschnittstelle aufweist.

4. Vorrichtung nach einem der vorhergehenden Ansprüche, **dadurch gekennzeichnet, daß** die Signalverarbeitung in Echtzeit erfolgt.

5. Vorrichtung nach einem der vorhergehenden Ansprüche, **dadurch gekennzeichnet, daß** diese bei Überschreiten des Belastungsgrenzwertes durch den Meßwert ein akustisches Signal abgibt.

6. Vorrichtung nach einem der vorhergehenden Ansprüche, **dadurch gekennzeichnet, daß** diese bei Überschreiten des Belastungsgrenzwertes durch den Meßwert ein sensorisches Signal abgibt.

7. Vorrichtung nach einem der vorhergehenden Ansprüche, **dadurch gekennzeichnet, daß** diese einen Meßwertspeicher aufweist.

8. Vorrichtung nach einem der vorhergehenden Ansprüche, **dadurch gekennzeichnet, daß** diese einen Speicher zur Hinterlegung eines zeitbezogenen Belastungsprofiles aufweist.

9. Vorrichtung nach einem der vorhergehenden Ansprüche, **dadurch gekennzeichnet, daß** die Datenverbindung drahtlos ausgebildet ist.

10. Vorrichtung nach einem der vorhergehenden Ansprüche, **dadurch gekennzeichnet, daß** diese eine Funktion aufweist, mittels welcher ein aktuell gemessener Meßwert als Belastungsgrenzwert eingesetzt wird.
